# EUROPEAN PATENT SPECIFICATION

(11) **EP 2 596 765 B1**
(45) Date of publication and mention of the grant of the patent: **04.01.2017**
(21) Application number: 11809437.4
(22) Date of filing: 15.07.2011
(51) Int. Cl.: A61F 2/95

(54) **STENT DEVICE**
STENTVORRICHTUNG
DISPOSITIF DE STENT

(30) Priority: 20.07.2010 JP 2010163376
(43) Date of publication of application: 29.05.2013
(73) Proprietor: Kyoto Medical Planning Co., Ltd., Kyoto-shi, Kyoto 607-8035 (JP)
(72) Inventor: IGAKI, Keiji, Kyoto-shi Kyoto 607-8035 (JP)
(74) Representative: Müller Hoffmann & Partner
(86) International application number: PCT/JP2011/004049
(87) International publication number: WO 2012/011261

(56) References cited:
- EP-A1- 0 697 226
- WO-A1-00/13737
- JP-A- 2002 531 219
- JP-A- 2004 523 303
- US-A- 5 116 318
- US-A- 5 591 222

## Description

### Technical field

This invention relates to a stent apparatus for holding a stent in contracted state with a stent covering member, wherein the stent is, when implanted into a vessel such as blood vessels, expanded in the diameter to scaffold the vessel from inside.

### Background Art

Heretofore, when stenosis occurs in a blood vessel, such as coronary artery, percutaneous transluminal coronary angioplasty (PTCA) employing stenting is performed in which the stenosed portion is expanded by using a medical balloon catheter, and a cylindrical stent is implanted into this expanded portion for scaffolding the vessel from inside so as to ensure the blood flow.

Stents used for this type of stenting have been proposed by the inventors in WO92/15342 (Patent Document 1), WO00/13737 (Patent Document 2) and WO2009/157164(Patent Document3). The stents proposed in these Patent Documents are made of cylindrically shaped polymer material whose shape is memorized to the size to scaffold blood vessels from the inside and, when implanted in the blood vessels, is self-expanded by heating with body temperature to the shape-memorized size. The stents expanded to the shape-memorized size keep self-expanding force to scaffold the blood vessels from inside.

Implantation of a stent into a blood vessel is conducted by using a catheter inserted into the blood vessel. At this time, the stent is mounted to the catheter and inserted into the blood vessel in contracted state having an outer diameter sufficiently smaller than the shape memorized size such that it can be smoothly inserted without injuring the inner wall of the blood vessel.

When implanted into the blood vessel, the stent formed by using a biodegradable polymer material is heated by body temperature; the diameter of the stent is expanded from the contracted state to the shape memorized size and the stent further keeps this expanded state by the self-expansion force of thistype of polymer material. It should be noted that, when the stent formed of the biodegradable polymer material is heated by body temperature and expanded to the shape memorized outer diameter, the diameter is gradually expanded over a sufficient period of time rather than immediately due to the nature of the material. This is because the biodegradable polymer material has a property as a viscoelastic body and viscous resistance is produced when it expands to the shape memorized size. As explained hereinabove, the expansion of the stent needs a certain length of time.

The stent formed by using a biodegradable polymer material is transported to an implantation position within the blood vessel, that is a lesion site, and then immediately expanded in diameter so that it is expanded to the size to scaffold the inner wall of the blood vessel by utilizing inflation force of a balloon capable of being rapidly expanded with injection of an expansion media.

The stent formed by using a biodegradable polymer material to be expanded by the above described balloon inflation force is mounted, in a contracted state, onto the balloon which has been mounted onto a tip of a catheter in a folded state, and is delivered to the implantation site together with this balloon. When transported to the desired implantation position in the blood vessel, the stent is implanted at the lesion site by supplying an expansion media into the balloon and rapidly expanding the stent to a size to scaffold the blood vessel from the inside. The once expanded stent formed by using a biodegradable polymer material keeps the shape memorized size by its self-expansion force even after the balloon is deflated by removal of the expansion medium, thereby scaffolding the implanted site from the inside to allow fluid path for blood in the blood vessel.

As stated above, the stent formed by using a biodegradable polymer material having shape memory property and being shape-memorized to the expanded size to scaffold the blood vessel from the inside in expanded state is heated by body temperature as it is inserted into the blood vessel, generating self-expansion force to recover the shape from the contracted state to expanded state. By the effect of this shape restoring function, the stent having crimped to the balloon in contracted state is expanded in diameter, thus making a gap between the stent and the balloon. For this reason, dislocation or disengagement might occur between the balloon and the stent mounted onto the balloon due to the friction force generated when the stent contacts with an inner wall of the blood vessel as it is inserted into the blood vessel. The stent disengaged from the balloon cannot be rapidly expanded in diameter with the balloon inflation force, making correct implantation to the intended site such as the lesion site impossible. The stent not disengaged from the balloon but just dislocated relative to the balloon may be subjected to the balloon inflation force unequally along its entire length, resulting in its unequal expansion along its entire length. The stent expanded unequally along its length cannot scaffold the vessel wall in the vessel as intended.

In order to solve the problem described above, the inventor of the present invention has proposed a stent delivery apparatus wherein a balloon catheter, having a balloon on which the stent is mounted, is inserted into a protective sheath (WO2004/103450:PLT4). Furthermore, in this stent delivery apparatus, a holding member holds one end of the stent to prevent stent dislocation relative to the balloon, when the stent is extruded from the protective sheath to be expanded in diameter.

### Citation List

### Patent Literature

PLT 1: WO92/15342
PLT 2: WO00/13737
PLT 3: WO2009/157164
PLT 4: WO2004/103450

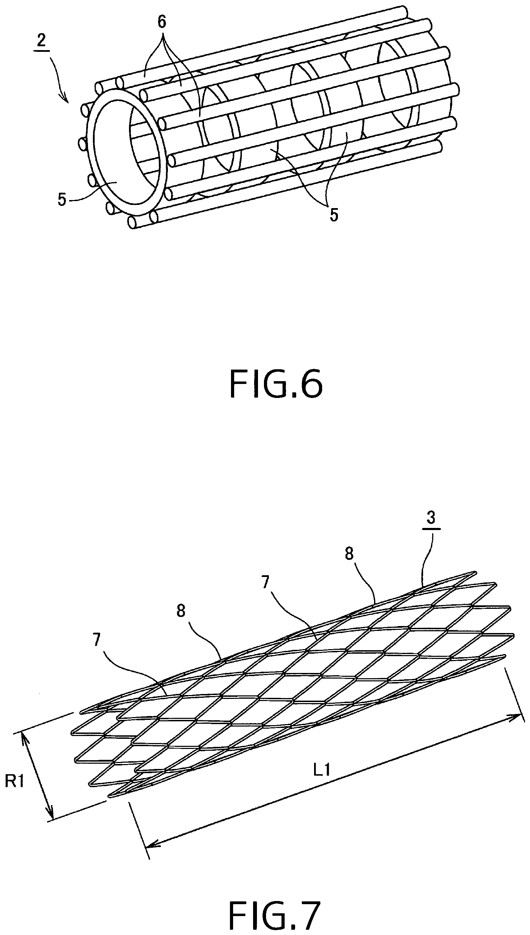

Furthermore, EP 0 697 226 A1 is directed to a stent device wherein a stent is fitted over the outer circumferential surface of a tubular cartridge which is radially expandable and contractible. The stent may be formed of a biodegradable poylmer. A sleeve may be fitted on at least one end portion of the tubular cartridge and an edge portion of the sleeve is fitted on an end portion of the stent for holding the stent on the tubular cartridge (cf. claim 4). The sleeve is e.g. a silicon resin and it is removed after the implantation of the stent as shown in Figure 6 and the description thereof (cf. col. 6,1. 2 et seq.).

US 5 591 222 A describes a device for dilating ducts in vivo, comprising a balloon-tipped catheter to which a cylindrical stent prepared by knitting or braiding or weaving biodegradable fibers to easily reduce diameter of the cylinder to a predetermined value is attached in a compressed condition. The stent is compressed by being inserted into a tube with a smaller diameter. The tube is made of polytetrafluorethylen (Teflon) or poypropylene (cf. col. 3, 1. 25 et seq.). In example 1 the tube (3) is a teflon tube (see col. 4, 1. 35 et seq.). Polytetrafluorethylene (PTFE, Teflon) and polypropylene are not biodegradable plastic materials.

### Summary of the Invention

### Technical Problem

In the above described stent delivery apparatus using the protective sheath, the outer diameter including the protective sheath accommodating the balloon catheter in which the stent is mounted on the balloon, must be large. For this reason, it is difficult to make the outer diameter of the balloon catheter small so as to configure a stent delivery apparatus capable of being used for a narrow blood vessel. Moreover, using the protective sheath results in a complex structure which is difficult to produce.

Furthermore, in the stent delivery apparatus using the protective sheath, operation for implanting a stent is cumbersome because, after the stent mounted on the balloon is inserted into the vicinity of the implantation site, the protective sheath must be advanced or recessed relative to the catheter so that the balloon protrudes from a tip of the protective sheath.

In addition, in the balloon catheters for transporting the above described stent into a vessel such as a blood vessel, shapes or flexibilities of catheters differ from one another and material properties or outer diameter contraction properties of the balloon are also of various extent. Operators, therefore, select an optimum stent in accordance with the lesion site and symptom thereof.

In a system using inflation force of a balloon to expand a stent, however, since types of balloon catheters used in accordance with types of stents are limited, optimum selection in accordance with a target site might be impossible.

One of the technical objects of the present invention is to provide a stent apparatus capable of preventing increase in the size of an apparatus used for transporting a stent to be expanded by balloon inflation to a lesion site in a site in a vessel.

Another technical object of the present invention is to provide a stent apparatus in which a stent to be expanded by balloon inflation can be easily mounted and securely positioned on any one of balloons of various shapes and structures of balloon catheters.

Another technical object of the present invention is to provide a stent apparatus in which a stent can be securely mounted and held on a balloon of various types of balloon catheters wherein the stent is formed of a biodegradable polymer material, shape-memorized to an expanded size larger than an inner diameter of a vessel when inserted into a vessel, expanded by balloon inflation to expanded state, and heated by body temperature to recover the shape.

Another technical object of the present invention is to provide a stent apparatus capable of holding a stent without preventing expansion of the stent by balloon inflation.

Another technical object of the present invention is to provide a stent apparatus for administrating drugs to a living body by implanting them with a stent so as to prevent thrombosis and intimal proliferation.

### Solution of Problem

To achieve the above-mentioned technical objects, the present invention provides a stent apparatus comprising: a tubular cartridge expandable and contractible in radial direction, the expansion of which in axial direction orthogonal to the radial direction being restricted; a stent formed of a biodegradable polymer as a tubular shape, shape memorized to an expanded size larger than an inner diameter of a target blood vessel in which the stent is to be implanted, and mounted to the tubular cartridge in contracted state; and a stent covering member formed of a biodegradable polymer having elasticity as a cylindrical shape having an inner diameter to keep the stent in contracted state by holding an outer surface of the stent and, when the stent is expanded when the stent is expanded to the shape memorized shape, plastically deformed to release the holding of the stent. In this stent apparatus, the stent is expanded together with the stent covering member along with expansion of the tubular cartridge in radial direction and released together with the stent covering member from the tubular cartridge by contraction of the tubular cartridge in radial direction.

Preferably, both of the stent covering member and the stent are formed of a biodegradable aliphatic polyester.

Specifically, the biodegradable polymer forming the stent covering member is preferably a copolymer of poly-L-lactide (PLLA) and poly-ε-caprolactone(PCL) having an elasticity in shape memorized state, the elastic force of which decreases as it expands from the expanded state.

More particularly, the biodegradable polymer forming the stent covering member is preferably a copolymer of poly-L-lactide (PLLA) and poly-ε-caprolactone(PCL) having a composition ratio of PLLA and PCL ranging from 95 to 20:5 to 80 in molar ratio (mol %).

The stent covering member may have a length covering whole of the outer surface of the stent. Preferably, the stent apparatus comprises a plurality of openings provided in the stent covering member.

One or both of the stent and the stent covering member may contain a drug. Preferably, the stent covering member contains a drug having intimal hyperplasia inhibiting efficacy and the stent contains a drug having antithrombotic efficacy.

### Advantageous Effects of Invention

Since the stent apparatus in said present invention holds a stent formed of a biodegradable polymer having self-expansion force working to recover a shape by gradually expanding from contracted state to shape memorized state by a stent covering member formed as an elastic cylinder having an inner diameter to hold the stent in contracted state, the stent can be held in contracted state on a balloon of a balloon catheter without using a protective sheath. The use of tubular cartridge for mounting the stent held in contracted state by the stent covering member on a balloon catheter allows arbitrary pairing of various types of stents and catheters, thus increasing degree of freedom of stent and catheter selection.

The stent covering member formed of a biodegradable polymer which is, when the stent is expanded to the shape memorized shape, plastically deformed to release the holding of the stent does not prevent expansion of the stent rapidly expanded together with the tubular cartridge by utilizing balloon inflation force. The once expanded stent covering member, therefore, will not elastically recovered to the contracted state, so that it does not prevent self-expansion of the stent formed of a biodegradable polymer expanded from the contracted state to the shape memorized size.

Furthermore, in the present invention, the stent held in contracted state by the stent covering member is expanded together with the tubular cartridge by the balloon and keeps the expanded state, while the tubular cartridge contracts along with balloon deflation, so that the stent in contracted state can be smoothly implanted in a predetermined indwelling position such as a lesion site in a vessel.

Forming the stent covering member with biodegradable aliphatic polyester having excellent biocompatibility can avoid adverse effect inflicted on a living body such as human body.

In addition, forming the stent covering member and the stent with same type of a biodegradable polymer achieves mutual affinity, assuring tighter contact between the stent and the stent covering member, so that the stent shape memorized to expanded state can be surely held in contracted state.

The stent covering material formed of a copolymer of poly-L-lactide (PLLA) and poly-ε-caprolactone(PCL) having a composition ratio of PLLA and PCL ranging from 95 to 20:5 to 80 in molar ratio (mol %) have improved elasticity and flexibility, thereby tightly holding a stent mounted together with a tubular cartridge on a balloon of a balloon catheter with the elasticity of the material, so that the stent and the tubular cartridge are tightly held on the balloon even if some outer force is applied during transportation within a vessel.

The stent covering member having a length covering whole of the outer surface of the stent can more precisely prevent disengagement or dislocation of the stent from the balloon.

Providing a plurality of openings provided in the stent covering member can prevent the inner wall of the blood vessel from being entirely covered, thereby ensuring blood flow to rami from blood vessel in which the stent is implanted.

Furthermore, implanting a stent covering member containing a drug having intimal hyperplasia inhibiting efficacy and/or antithrombotic efficacy together with a stent into a living body vessel enables administration of drugs to a living body, thereby preventing intimal proliferation and/or thrombosis.

Besides, in addition to the stent covering member, the stent may also contain a drug, so that different types of drugs can be simultaneously administered.

### Brief Description of the Drawings

(Fig.1) Fig.1 is a perspective view of an assembly of a stent apparatus according to the present invention.
(Fig.2) Fig.2 is a perspective view of a stent apparatus showing its appearance.
(Fig.3) Fig.3 is a perspective view of a tubular cartridge constituting a stent apparatus according to the present invention.
(Fig.4) Fig.4 is a front view of another example of a tubular cartridge.
(Fig.5) Fig.5 is a front view of yet another example of a tubular cartridge.
(Fig.6) Fig.6 is a perspective view of yet another example of a tubular cartridge.
(Fig.7) Fig.7 is a perspective view of a stent expanded to a size for implantation into a blood vessel.
(Fig.8) Fig.8 is a cross sectional view of a stent apparatus showing a state in which the stent is held in a contracted state by stent covering member.
(Fig.9) Fig.9 is a characteristic diagram showing a relationship between elastic property of a stent covering member and expanded state of a stent.
(Fig.10) Fig.10 is a schematic side view showing a using method of a stent apparatus.
(Fig. 11) Fig. 11 is a side view of a slider as an insertion assist tool used for mounting a tubular cartridge onto a balloon.
(Fig. 12) Fig.12 is a perspective view of another example of a slider.
(Fig.13) Fig.13 is a perspective view of yet another example of a slider.
(Fig.14) Fig.14 is a perspective view of yet another example of a slider.
(Fig.15) Fig.15 is a schematic side view showing how to mount a stent apparatus on to a balloon provided on catheter.
(Fig. 16) Fig. 16 is a schematic cross sectional view of an example in which a tubular cartridge has a taper.
(Fig.17) Fig.17 is a side view showing another embodiment of a stent apparatus.
(Fig. 18) Fig. 18 is a perspective view of another example of a stent covering member.
(Fig.19) Fig.19 is a perspective view of yet another example of a stent covering member.

### Description of Embodiments

Referring to attached drawings, embodiments of the present invention is described hereinafter.

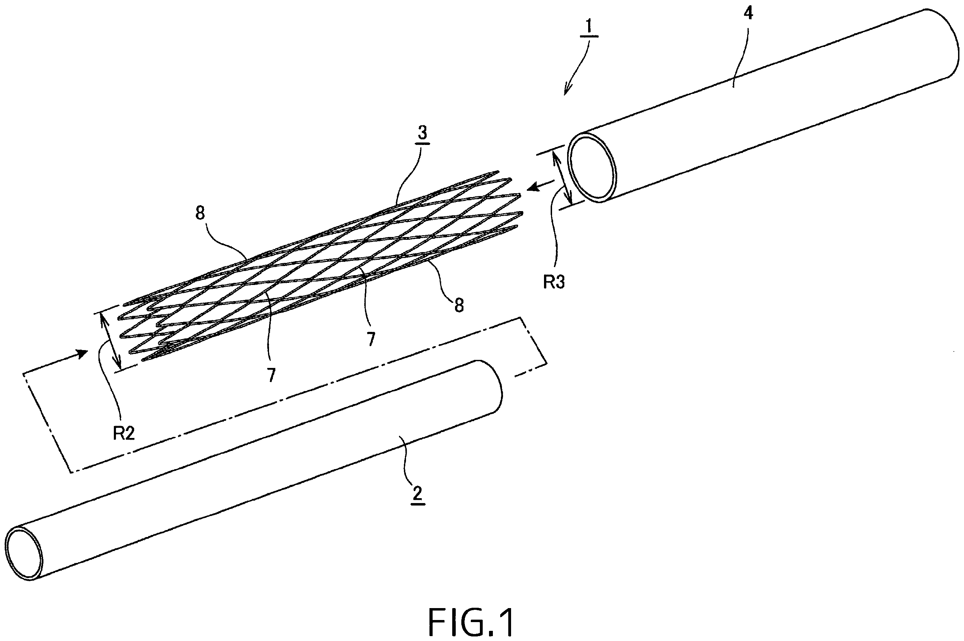

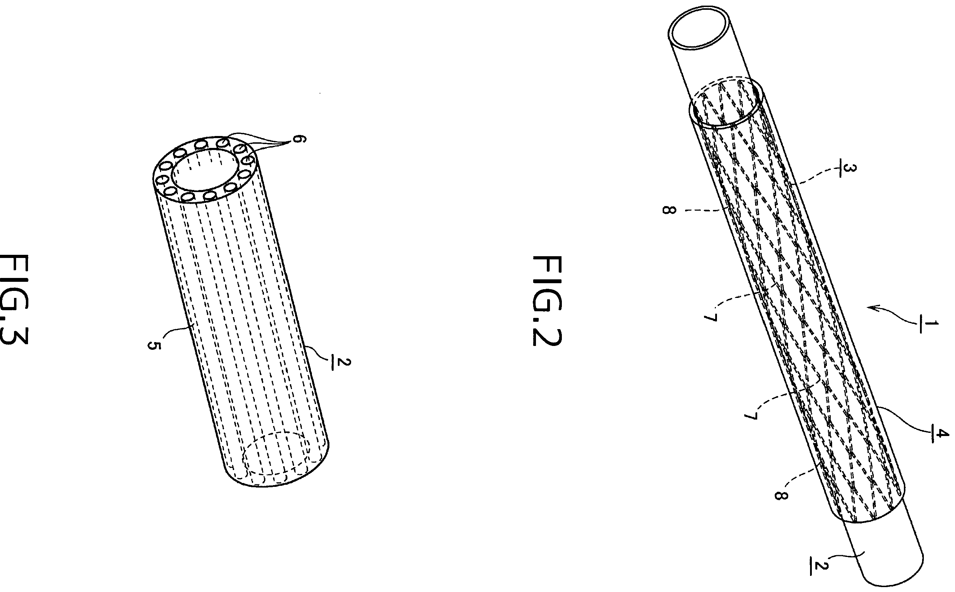

As shown in Figures 1 and 2, a stent apparatus 1 according to the present invention includes a tubular cartridge 2 having an appropriate length, a cylindrical stent 3 mounted on an outer surface of this tubular cartridge 2 and a stent covering member 4 covering outer side of the stent 3 to hold the stent 3.

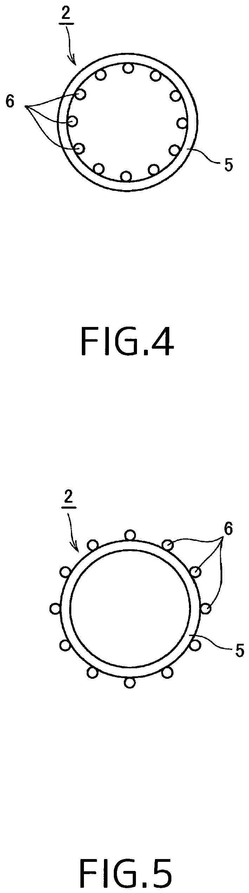

The tubular cartridge 2 constituting the stent apparatus 1 of the present invention is preferably formed of a material easily expandable in radial direction and hardly expandable in axial direction. For example, this tubular cartridge 2 is formed by incorporating a strand 6 of a yarn having certain rigidity into a tube 5 having the expandability such that the strand 6 extends in axial direction. By employing this constitution, the strand 6 restricts the axial expansion of the tubular cartridge 2. Moreover, the tubular cartridge 2 easily expandable in radial direction and hardly expandable in axial direction may be formed by adhering the strand 6 to the inner surface of the tube 5, as shown in figure 4, or by adhering the strand 6 to the outer surface of the tube 5, as shown in figure 5.

In this tubular cartridge 2, the section expandable in radial direction may be entire cartridge 2 or may be a part of it.

A tubular cartridge 2 shown in figure 6 is an example in which the tube 5 expandable in radial direction is partly provided in longitudinal direction of the strand 6. By employing this type of constitution, the tubular cartridge 2 would have low expandability in axial direction, high flexibility in axial direction and excellent trackability to track a bent or curved vessel such as blood vessel. When employing this type of constitution, tube 5 and the strand 6 is preferably formed of same kind of material so as to realize good adhesion. If, for example, the tube 5 is formed of a polymer material, then it is preferable that the strand 6 is also formed of the polymer material.

The stent 3 mounted on the tubular cartridge 2 constituted as stated above is now explained. As shown in figures 1 and 7, for example, this stent 3 is is constituted as a cylindrical body by bending a continuous strand 7 in zigzag design such that linear parts and bend parts alternate in sequence and combining tubular body forming elements 8 to form a single channel from one side to the other. The dimensions of this stent 3 are appropriately selected in accordance with a living body vessel such as a blood vessel in which the stent 3 is implanted. For example, as shown in figure 7, the stent 3 constituted to be implanted in a blood vessel such as an artery is tubularly shaped wherein the outer diameter R1 is formed to be 2 to 5 mm and the length L1 is formed to be 10 to 40 mm as for the diameters when implanted in the blood vessel.

It should be noted that the dimensions of the stent 3 is appropriately selected in accordance with a blood vessel into which the stent 3 is implanted. Particularly, the stent 3 is formed to have an outer diameter to scaffold the blood vessel from the inside in which the stent 3 is implanted.

Furthermore, the stent 3 is formed of a biodegradable polymer so as not to exert adverse effect on a living body when implanted into a living body such as a human body. For example, polylactic acid (polylactide:PLA), polyglycolic acid (PGA), polyglactin (copolymer of polyglycolic acid and polylactic acid), polydioxanone, polyglyconate (copolymer of trimethylene carbonate and glycolide), and copolymer of polyglycolic acid or polylactic acid and poly-ε-caprolactone can be used for this biodegradable polymer. Two or more of these materials can be compounded and used for this biodegradable polymer. In view of safety to living bodies, it is desirable to use poly-L-lactide (PLLA) for this biodegradable polymer.

In the stent 3 formed by bending the continuous strand 7 in zigzag design such that linear parts and bend parts alternate in sequence and combining the tubular body forming elements 8, varying bend angles of the strand 7 results in expanded state having larger outer diameter or contracted state having smaller outer diameter.

As shown in figure 7, the stent 3 formed by bending the continuous strand 7 in zigzag design is shape memorized to the expanded state in which the bend parts are opened to make the bend angles larger. The size to which the stent 3 is shape memorized is a size enough to scaffold a blood vessel from the inside when implanted in the blood vessel.

The stent 3 shape memorized to the size to scaffold the blood vessel when implanted in the blood vessel as stated above is contracted to a size capable of being inserted into the blood vessel, as shown in figure 1. This contraction is conducted by applying a pressure to the outer peripheral of the tubularly formed stent 3. For example, this contracted state is achieved by compressing the stent 3 into contracted state and insert it into a tubular shaped mold having an inner diameter corresponding to the contracted size.

After the stent 3 formed of a biodegradable polymer and shape memorized to a certain size is contracted by applying a pressure to the bend portions to make the outer diameter smaller, applying a heat equal to or close to the shape memory temperature produces self-expansion force to open the contracted bend parts to recover the shape memorized size. That is, when this stent 3 is inserted into a living body and heated by body temperature, self-expansion force works to gradually expand the diameter from the contracted state to the shape memorized state. When this self-expansion force works to expand the diameter, dislocation or disengagement might occur between the stent 3 and the tubular cartridge 2 holding the stent 3. For example, when the stent 3 is mounted on a balloon via the tubular cartridge 2 and transported through a blood vessel, expansion of the stent 3 might trigger dislocation against the tubular cartridge 2 or the balloon, or disengagement from the balloon due to the friction force generated when the stent 3 contacts with an inner wall of the blood vessel.

For this reason, the stent covering member 4 is used to keep the stent 3 in contracted state, which would otherwise recover to the shape memorized size if a heat equal to or close to the shape memory temperature produces the self-expansion force. As shown in figure 1, this stent covering member 4 is shaped as a cylinder having an inner diameter R3 smaller than the outer diameter R2 of the contracted stent 3 so as to be able to keep the compressed and contracted stent 3 in the contracted state.

The stent covering member 4 is formed of a biodegradable polymer initially having a certain elasticity and capable of plastic deformation by a certain amount of expansion. This stent covering member 4 formed of a biodegradable polymer has an elasticity in the cylindrical state having an inner diameter R3 smaller than the outer diameter R2 of the contracted stent 3 and is plastically deformed by expansion of the diameter from this size.

In this embodiment, the stent covering member 4 is formed of an aliphatic polyester as a biodegradable polymer initially having elasticity and capable of plastic deformation by a certain amount of expansion as explained above. More particularly, this material is a copolymer of poly-L-lactide (PLLA) and poly-ε-caprolactone(PCL).

As shown in figure 2, the stent 3 is contracted from the shape memorized size to the contracted size, mounted to the outer surface of the tubular cartridge 2 and inserted into the stent covering member 4 together with this tubular cartridge 2. Here, , the stent 3 is elastically held by the stent covering member 4 having the inner diameter R3 smaller than the contracted outer diameter R2, thereby tightly mounted on the outer surface of the tubular cartridge 2 while keeping the contracted state.

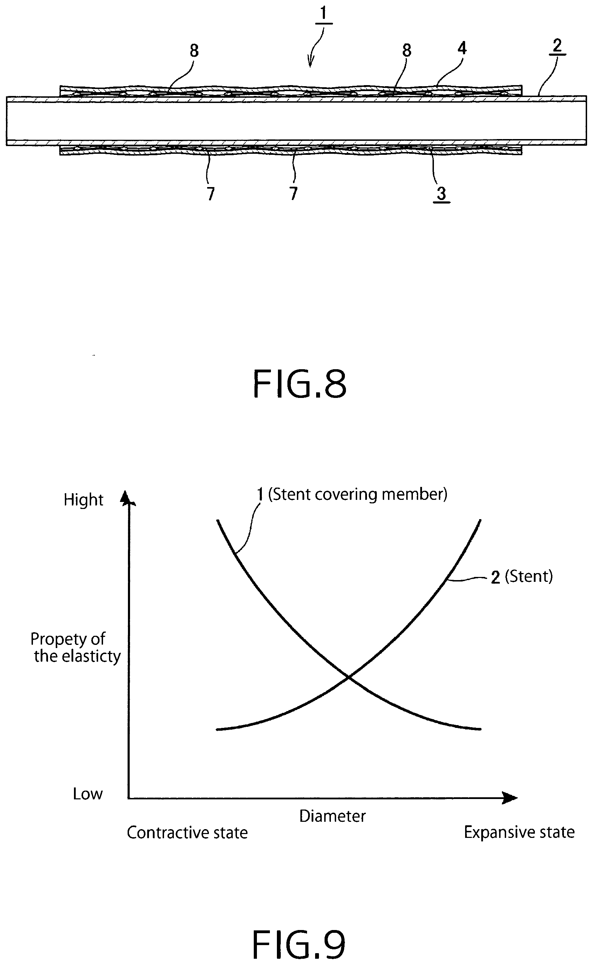

The stent covering member 4 formed of a copolymer of poly-L-lactide (PLLA) and poly-ε-caprolactone(PCL) and shaped as a cylinder having the inner diameter R3 smaller than the contracted outer diameter R2 of the stent 3 has a property in which the elasticity decreases as the diameter becomes larger from the initial state, as shown in figure 9. The stent apparatus 1 according to the present invention, therefore, mount the stent 3 in contracted state onto the tubular cartridge 2 utilizing the fact that the elasticity of the stent covering member 4 is larger than the self-recovery force of the stent 3 at the initial state in which the stent covering member 4 holds the stent 3 in contracted state as assumed obvious from the characteristic diagram shown in figure 9.

### in figure 9.

Since the stent covering member 4 loses the elasticity and is plastically deformed without recovering to the initial size when expansion rate exceeds a certain level, the stent covering member 4 decreases the elasticity and is plastically deformed. The stent covering member 4 having this property is plastically deformed without elastic recovery when the stent 3 mounted together with the tubular cartridge 2 onto the balloon of the balloon catheter is expanded beyond a certain amount by the expansion force of the balloon. When the stent covering member 4 is expanded to the extent capable of plastic deformation, the expansion force of the stent 3 by heating exceeds the elastic recovery force of the stent covering member 4. When the stent 3 is further heated by body temperature, the stent 3 is then expanded to the shape memorized size by the self-recovery force larger than the recovery force of the covering member 4.

In the stent apparatus 1 according to the present invention, since the inner diameter of the stent covering member 4 is formed to a size for holding the stent 3 in the contracted state, thereby keeping the stent 3, which is shape memorized to the expanded state, in contracted state together with the tubular cartridge 2, dislocation or disengagement against the tubular cartridge 2 or the balloon of the catheter on which the tubular cartridge 2 is mounted can be prevented during insertion into a blood vessel. The stent covering member 4 is plastically deformed by expanding it by a certain amount from the initial size to hold the stent 3 in contracted state, thereby enabling the shape memorized stent 3 to expand to expanded size, so that the stent 3 can expand and scaffold the blood vessel at a correct implantation position in the blood vessel.

It should be noted that the stent covering member 4 is preferably formed of the same type of the biodegradable polymer as the stent 3 held by this stent covering member 4. This is because the excellent affinity will assure tighter contact between the stent 3 and the stent covering member 4, so that the stent 3 shape memorized to expanded state can be secured in contracted contracted state.

For this reason, in the present embodiment, both of the stent 3 and the stent covering member 4 are formed of aliphatic polyester. More particularly, the stent 3 is formed of poly-L-lactide (PLLA) and the stent covering member 4 is formed of a copolymer of poly-L-lactide (PLLA) and poly-ε-caprolactone(PCL).

As shown in figure 2, the stent 3 held by the stent covering member 4 in contracted state is mounted on the outer surface of the tubular member together with the stent covering member 4. The stent 3 and the stent covering member 4 mounted on the outer surface of the tubular cartridge 2 is mounted together with the tubular cartridge 2 on a balloon of a catheter, and transported together with the catheter and the tubular cartridge 2 through a curved or serpentine blood vessel to a lesion site. Similar to the tubular cartridge 2, it is therefore necessary for the stent 3 and the stent covering member 4 mounted on the tubular cartridge 2 to have enough flexibility to pass the curved and serpentine blood vessel and holding ability to hold the stent on the balloon.

PLLA is generally a hard and fragile material. If the stent covering member 4 is formed only of PLLA, since this material does not have enough flexibility, it might be folded in the curved and serpentine blood vessel, the both ends of the stent covering member 4 contacting with the inner wall of a blood vessel might injure the blood vessel, or friction with the inner wall might trigger dislocation or disengagement of the stent 3 and the stent covering member against the tubular cartridge 2.

In order to solve the problems described above, introducing PCL into PLLA can add elastic property to the PLLA, thereby improving elasticity and flexibility of the material. For example, adding 5 mol% of PCL to PLLA can reduce bending stiffness and extremely improve elasticity and flexibility. Furthermore, adding 10 mol% of PCL to PLLA can assure elasticity and flexibility in the temperature region around 37 degrees Celsius such that the the stent covering member can keep sufficient elasticity to tightly hold the stent 3 in contracted state on the tubular cartridge 2.

In the stent covering member 4 formed of a copolymer of PLLA and PCL, increasing the composition ratio of the PCL will decrease the strength and elastic modulus and increase rupture elongation. The stent covering member 4 formed only of PCL is easily elongated and ruptured by smaller force. On the other hand, if 80 to 90 mol% of PCL is added to PLLA, melting temperature might drop to around human body temperature. If the melting temperature of the stent covering member 4 is around human body temperature or lower, the stent covering member 4 will soften or melt in a blood vessel and consequently it cannot sufficiently hold the stent 3.

For the reasons stated above, the biodegradable polymer material constituting the stent covering member 4 is desirably a copolymer of PLLA and PCL wherein the composition ratio of PLLA and PCL ranges from 95 to 20:5 to 80 in molar ratio (mol %).

More preferably, the biodegradable polymer material constituting the stent covering member 4 is a copolymer of PLLA and PCL wherein the composition ratio of PLLA and PCL ranges from 75 to 25:25 to 75 in molar ratio (mol %). The composition ratio of PLLA and PCL ranging from 75 to 25:25 to 75 in molar ratio (mol %) can realize strength and elasticity sufficient to secure the stent 3 in contracted state while keeping the melting temperature at which no melting occurs.

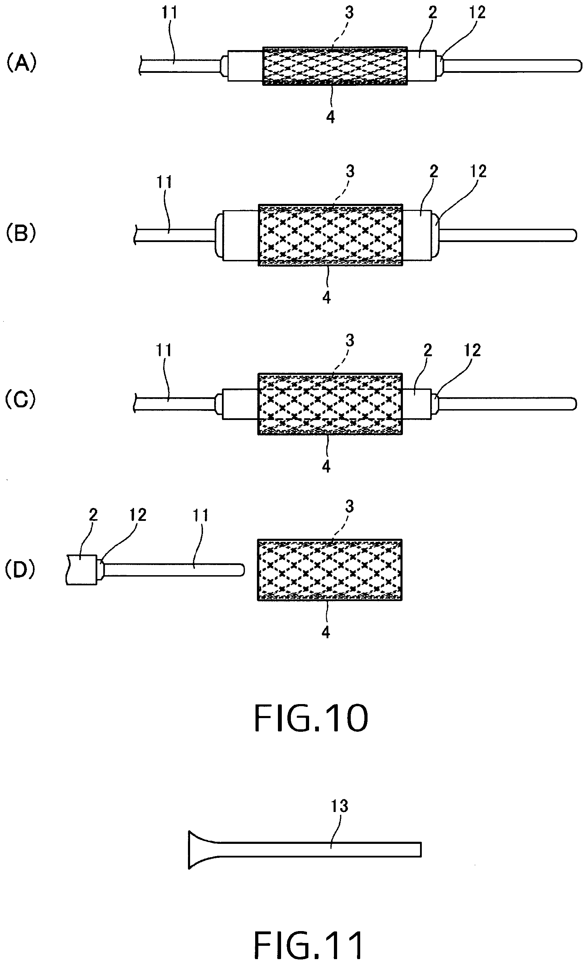

A method for using the above described stent 1 is now explained. In this embodiment, as shown in figure 10(A), the stent 3 held in the stent covering member 4 in contracted state and mounted on the outer surface of the tubular cartridge 2 is mounted together with the tubular cartridge 2 on a balloon 12 provided on a catheter 11. The stent 3 in this state is transported to a lesion site in a vessel and, after arrival at the lesion site, expanded by supplying an expansion media into the balloon 12 as shown in figure 10 (B). When the balloon 12 is inflated, the tubular cartridge 2, the stent 3 and the stent stent covering member 4 are also expanded in radial direction along with this balloon inflation.

Next, as shown in figure 10(C), the balloon 12 is deflated by drawing the expansion medium from the balloon 12. At this time, although the tubular cartridge 2 is contracted along with the balloon 12, the stent 3 and the stent covering member 4 keep their expanded state. Subsequently, as shown in figure 10(D), the catheter 11 is drawn from the lesion site in which the stent 3 has been implanted. The tubular cartridge 2 is hereby removed together with the balloon 12 of the catheter 11 while the stent 3 stays together with the stent covering member 4 at the lesion site, thereby completing implantation in the blood vessel.

As stated above, the stent apparatus 1 of the present invention is used by mounting it on the balloon 12 provided at the catheter 11. It is therefore desirable that the stent apparatus 1 be quickly mounted on the balloon 12 of the catheter 11 at operation.

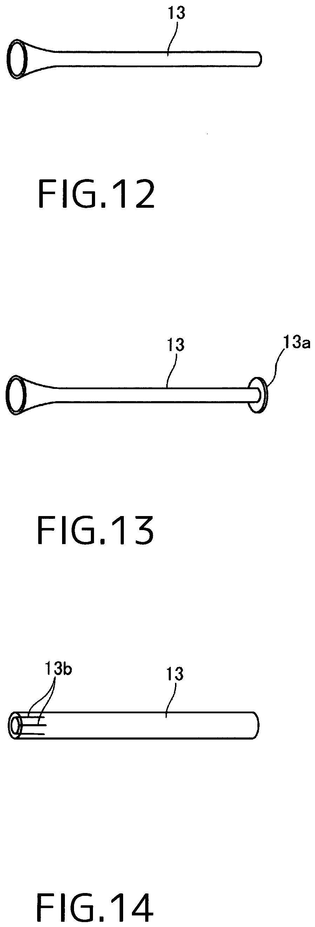

Because of this, a taper needs be provided at an opening of the tubular cartridge 2 or an insertion assist tool of some kind needs to be used for mounting on the balloon 12. This insertion assist tool may be a thin line-shaped guide wire or a non-uniform diameter body (hereinafter called a slider) having gradually increased diameter. Figures 11 to 14 show various sliders wherein figure 11 shows a rod-shaped solid type slider 13, figure 12 shows a hollow type slider 13, figure 13 shows a slider 13 having a stopper 13a at one end and figure 13 shows a slider 13 having a scratch 13b at one end.

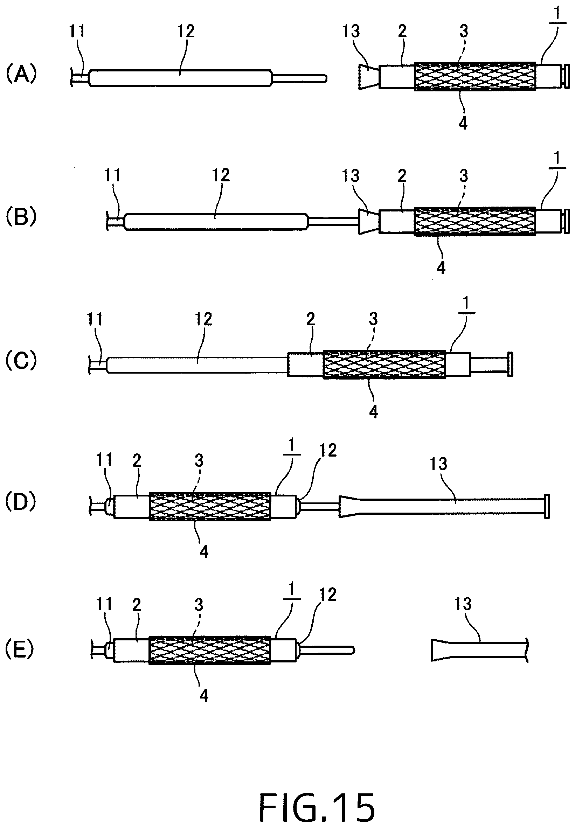

When mounting the stent apparatus, firstly, the narrower end of the slider 13 is inserted into one end of the tubular cartridge 2 as shown in figure 15(A). Subsequently, as shown in figure 15(B), a tip end of the catheter 11 is inserted into the wider end of the slider 13, and then, as shown in figure 15(C), the tubular cartridge 2 is moved together with the stent 3 along the slider 13.

When the tubular cartridge 2 arrives at the balloon 12 of the catheter 11 as shown in figure 15(D), removing the slider 13 completes the mounting of the stent apparatus 1 onto the balloon 12 as shown in figure 15(E).

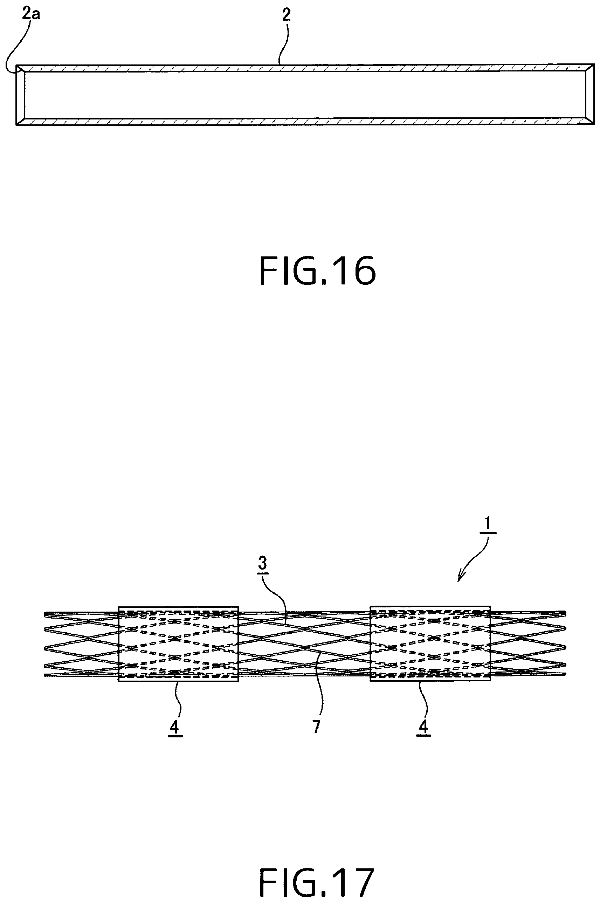

Alternatively, in place of using the above stated insertion assist tool, as shown in figure 16, a taper section 2a may be provided at the inner surface of at least one end of the tubular cartridge 2, thereby facilitating the mounting onto the catheter 11 and the balloon 12.

Although the above described embodiment illustrates the stent covering member 4 formed to cover whole of the outer surface of the stent 3 so as to prevent dislocation or disengagement of the stent 3 against the tubular cartridge 2 when transported in a curved and serpentine blood vessel, this invention is not limited to this embodiment. The stent covering member 4 may be of any shape as long as it can hold the stent 3 while preventing dislocation or disengagement against the tubular cartridge 2. For example, as shown in figure 17, the stent covering member 4 may be constituted so as to hold a part of the outer surface of the stent 3. In this case, a plurality of stent covering members 4 may be used to hold the stent 3.

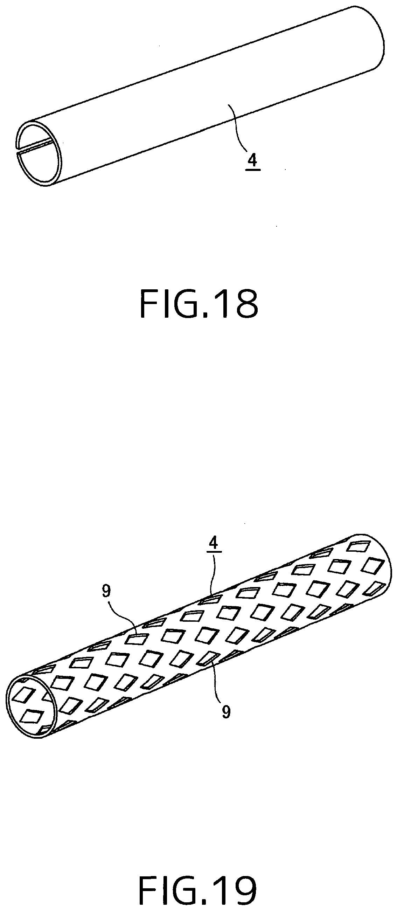

Moreover, the stent covering member 4 may be a biodegradable polymer material sheet formed as a cylinder. The stent covering member 4 may be of any shape as long as it can hold the stent 3 shape memorized to an expanded size larger than an inner diameter of a target blood vessel in which the stent is to be implanted and keep this stent 3 in contracted state. For example, the stent covering member 4 may be a shape as shown in figure 18 having a C-shaped cross section.

It should be noted that, a blood vessel in which the stent 3 is to be implanted has sections from which a plurality of rami branch off. If the stent 3 is implanted at these sections, there is a risk that the stent 3 or the stent covering member 4 will obstruct the entire surface of the inner wall of the blood vessel, thus blocking blood flow to the rami. In order to solve the above problem, problem, as shown in figure 19, a plurality of openings 9 are provided in the stent covering member 4 of the present embodiment to ensure blood flow from main blood vessel to rami. The openings 9 therefore can be of any shape including circular and rectangular shapes as long as they can ensure blood flow to rami. It is desirable that the openings 9 be provided and uniformly distributed on the entire surface of the stent covering member 4.

Furthermore, the biodegradable polymer constituting the stent covering member 4 may contain a drug. The stent apparatus 1 according to the present invention can be used as a drug-eluting stent carrying a drug by constituting the stent covering member 4 with a biodegradable polymer containing the drug.

By using a drug having antithrombotic efficacy or intimal hyperplasia inhibiting efficacy as a drug contained in the biodegradable polymer thrombotic, which is said to occur due to stent implantation, can be prevented or intimal hyperplasia can be inhibited.

In the stent apparatus 1 according to the present invention using the above described stent covering member 4, the stent 3 can also contain a drug. The stent 3 can contain a drug by constituting the stent 3 with a biodegradable polymer containing the drug. Alternatively, a biodegradable polymer solution containing a drug may be applied to a surface of the stent 3. In this case, different types of drugs can be simultaneously administered by differentiating the drug contained in the stent 3 from the drug contained in the stent covering member 4.

Furthermore, this invention is not limited to the stent formed by bending the continuous strand 7 and combining the tubular body elements 8, but can be widely applied to stents formed of a biodegradable polymer as a cylindrical shape and shape memorized to an expanded size larger than an inner diameter of a target blood vessel in which the stent is to be implanted.

As is obvious from the above description, since the present invention uses the stent in a state covering the tubular cartridge expandable in radial direction, operators can arbitrarily chose the catheter and the stent.

In addition, since the stent apparatus according to the present invention can be mounted not only on balloon catheters for post-dilatation, but also on balloon catheters for pre-dilatation, thereby enabling reuse of balloon catheters, a significant economic advantage can be achieved.

Furthermore, the stent apparatus according to the present invention allows the stent to be handled in compact fashion, so that excessive labor of operators or assistants putting on rubber gloves can be avoided.

### Reference Signs List

- 1: stent apparatus
- 2: tubular cartridge
- 3: stent
- 4: stent covering member

## Claims

1. A stent apparatus (1) comprising:
a tubular cartridge (2) expandable and contractible in radial direction, the expansion of which in axial direction orthogonal to the radial direction being restricted; and
a stent (3) formed of a biodegradable polymer as a tubular shape, shape memorized to an expanded size larger than an inner diameter of a target blood vessel in which the stent (3) is to be implanted, and mounted to the tubular cartridge (2) in contracted state;
**characterized in that**
a stent covering member (4) formed of a biodegradable polymer is present having elasticity as a cylindrical shape having an inner diameter to keep the stent (3) in contracted state by holding an outer surface of the stent (3) and, when the stent (3) is expanded to the shape memorized shape, plastically deformed to release the holding of the stent (3), wherein
the stent (3) is expanded together with the stent covering member (4) along with expansion of the tubular cartridge (2) in radial direction and released together with the stent covering member (4) from the tubular cartridge (2) by contraction of the tubular cartridge (2) in radial direction.

2. The stent apparatus (1) according to claim 1, wherein both of the stent covering member (4) and the stent (3) are formed of a same type of biodegradable polymer.

3. The stent apparatus (1) according to claim 1, wherein both of the stent covering member (4) and the stent (3) are formed of a biodegradable aliphatic polyester.

4. The stent apparatus (1) according to claim 1, wherein the biodegradable polymer forming the stent covering member (4) is a copolymer of poly-L-lactide (PLLA) and poly-ε-caprolactone(PCL) having a composition ratio of PLLA and PCL ranging from 95 to 20:5 to 80 in molar ratio (mol %).

5. The stent apparatus (1) according to one of claims 1 to 4, wherein the stent covering member (4) has a length covering whole of the outer surface of the stent (3).

6. The stent apparatus (1) according to one of claims 1 to 5, further comprising a plurality of openings (9) provided in the stent covering member (4).

7. The stent apparatus (1) according to one of claims 1 to 6, wherein one or both of the stent (3) and the stent covering member (4) contain a drug.

8. The stent apparatus (1) according to one of claims 1 to 6, wherein the stent covering member (4) contains a drug having intimal hyperplasia inhibiting efficacy and the stent (3) contains a drug having antithrombotic efficacy.

## Patentansprüche

1. Stentvorrichtung (1), umfassend:
eine in einer radialen Richtung expandierbare und kontrahierbare röhrenförmige Patrone (2), deren Expansion in einer zur radialen Richtung orthogonalen axialen Richtung beschränkt ist; und
einen Stent (3), der aus einem biologisch abbaubaren Polymer als eine Röhrenform ausgebildet ist, mit einem Formgedächtnis für eine expandierte Größe, die größer als ein Innendurchmesser eines Ziel-Blutgefäßes ist, in das der Stent (3) implantiert werden soll, und im kontrahierten Zustand an der röhrenförmigen Patrone (2) montiert wird;
**dadurch gekennzeichnet, dass**
ein aus einem biologisch abbaubaren Polymer bestehendes, einen Stent bedeckendes Element (4) mit einer Elastizität als eine zylindrische Form mit einem Innendurchmesser vorhanden ist, um den Stent (3) durch Halten einer äußeren Oberfläche des Stents (3) im kontrahierten Zustand zu halten, und, wenn der Stent (3) zu der Form gemäß Formgedächtnis expandiert wird, plastisch verformt wird, um das Halten des Stents (3) zu lösen, wobei
der Stent (3) zusammen mit dem einen Stent bedeckenden Element (4) einhergehend mit einer Expansion der röhrenförmigen Patrone (2) in radialer Richtung expandiert und durch Kontraktion der röhrenförmigen Patrone (2) in radialer Richtung zusammen mit dem einen Stent bedeckenden Element (4) von der röhrenförmigen Patrone (2) gelöst wird.

2. Stentvorrichtung (1) nach Anspruch 1, wobei sowohl das einen Stent bedeckende Element (4) als auch der Stent (3) aus einer gleichen Art von biologisch abbaubarem Polymer geschaffen sind.

3. Stentvorrichtung (1) nach Anspruch 1, wobei sowohl das einen Stent bedeckende Element (4) als auch der Stent (3) aus einem biologisch abbaubaren aliphatischen Polyester geschaffen sind.

4. Stentvorrichtung (1) nach Anspruch 1, wobei das biologisch abbaubare Polymer, welches das einen Stent bedeckende Element (4) bildet, ein Copolymer von Poly-L-Laktid (PLLA) und Poly-ε-Caprolacton (PCL) mit einem Zusammensetzungsverhältnis von PLLA und PCL, das von 95 bis 20 : 5 bis 80 im Molverhältnis (Mol-%) reicht, ist.

5. Stentvorrichtung (1) nach einem der Ansprüche 1 bis 4, wobei das einen Stent bedeckende Element (4) eine die gesamte äußere Oberfläche des Stents (3) bedeckende Länge aufweist.

6. Stentvorrichtung (1) nach einer der Ansprüche 1 bis 5, ferner umfassend eine Vielzahl von Öffnungen (9), die in dem einen Stent bedeckenden Element (4) vorgesehen sind.

7. Stentvorrichtung (1) nach einem der Ansprüche 1 bis 6, wobei der Stent (3) oder das einen Stent bedeckende Element (4) oder beide ein Medikament enthalten.

8. Stentvorrichtung (1) nach einem der Ansprüche 1 bis 6, wobei das einen Stent bedeckende Element (4) ein Medikament mit einer Intimahyperplasie hemmenden Wirksamkeit enthält und der Stent (3) ein Medikament mit einer antithrombotischen Wirkung enthält.

## Revendications

1. Dispositif pour stent (1) comprenant:
une cartouche tubulaire (2) apte à être dilatée et contractée dans le sens radial, la dilatation de ladite cartouche dans le sens axial orthogonal par rapport au sens radial étant limitée ; et
un stent (3) qui se compose d'un polymère biodégradable de forme tubulaire, cette forme étant mémorisée à une taille dilatée plus grande qu'un diamètre intérieur d'un vaisseau sanguin ciblé dans lequel le stent (3) doit être implanté, et qui est monté dans la cartouche tubulaire (2) à l'état contracté;
**caractérisé**
**en ce qu'**il est prévu un élément de recouvrement de stent (4) qui se compose d'un polymère biodégradable, qui présente une élasticité, qui a une forme cylindrique avec un diamètre intérieur pour maintenir le stent (3) à l'état contracté en tenant la surface extérieure dudit stent (3), et qui, quand le stent (3) est dilaté jusqu'à la forme mémorisée, est déformé plastiquement pour relâcher la tenue du stent (3),
le stent (3) se dilatant avec l'élément de recouvrement de stent (4) en même temps que la dilatation de la cartouche tubulaire (2) dans le sens radial, et étant dégagé, avec ledit élément de recouvrement de stent (4), de la cartouche tubulaire (2) par contraction de celle-ci dans le sens radial.

2. Dispositif pour stent (1) selon la revendication 1, dans lequel l'élément de recouvrement de stent (4) et le stent (3) se composent tous les deux d'un même type de polymère biodégradable.

3. Dispositif pour stent (1) selon la revendication 1, dans lequel l'élément de recouvrement de stent (4) et le stent (3) se composent tous les deux d'un même type de polyester aliphatique biodégradable.

4. Dispositif pour stent (1) selon la revendication 1, dans lequel le polymère biodégradable qui forme l'élément de recouvrement de stent (4) est un copolymère de poly-L-lactide (PLLA) et de poly-ε-caprolactone (PCL) qui présente un rapport de composition de PLLA et PCL allant de 95 à 20:5 à 80 en rapport molaire (% en moles).

5. Dispositif pour stent (1) selon l'une des revendications 1 à 4, dans lequel l'élément de recouvrement (4) présente une longueur qui couvre toute la surface extérieure du stent (3).

6. Dispositif pour stent (1) selon l'une des revendications 1 à 5, comprenant également plusieurs ouvertures (9) prévues dans l'élément de recouvrement de stent (4).

7. Dispositif pour stent (1) selon l'une des revendications 1 à 6, dans lequel le stent (3) ou l'élément de recouvrement de stent (4), ou les deux, contiennent un médicament.

8. Dispositif pour stent (1) selon l'une des revendications 1 à 6, dans lequel l'élément de recouvrement de stent (4) contient un médicament à action anti-hyperplasie de l'intima, et le stent (3) contient un médicament à action antithrombotique.
